Europäisches Patentamt

European Patent Office

Office européen des brevets

(19)

(11) Veröffentlichungsnummer: **0 339 389 B1**

(12) **EUROPÄISCHE PATENTSCHRIFT**

(45) Veröffentlichungstag der Patentschrift: **22.09.93**

(51) Int. Cl.⁵: **G01N 33/543**, G01N 33/569

(21) Anmeldenummer: **89106688.8**

(22) Anmeldetag: **14.04.89**

(54) **Diagnose-Hilfsmittel.**

(30) Priorität: **25.04.88 CH 1537/88**
**02.05.88 CH 1635/88**

(43) Veröffentlichungstag der Anmeldung:
**02.11.89 Patentblatt 89/44**

(45) Bekanntmachung des Hinweises auf die
Patenterteilung:
**22.09.93 Patentblatt 93/38**

(84) Benannte Vertragsstaaten:
**AT BE CH DE FR GB IT LI NL SE**

(56) Entgegenhaltungen:
**EP-A- 0 150 844**
**EP-A- 0 173 629**
**EP-A- 0 174 853**
**US-A- 4 663 277**

**ANALYTICAL BIOCHEMISTRY, Band 142, Nr. 1, Oktober 1984 C.F. MEARES et al. "Conjugation of Antibodies with Bi- functional Chelating Agents: Isothiocyanate and Bromacetamide Reagents, Methods of Analysis, and Subsequent Addition of Metal Ions" Seiten 68-78**

**E.Hochuli; et al., Journal of Chromatography, 411 p.177-184 (1987)**

(73) Patentinhaber: **F. HOFFMANN-LA ROCHE AG**
**Postfach 3255**
**CH-4002 Basel(CH)**

(72) Erfinder: **Döbeli, Heinz, Dr.**
**Lupsingerstrasse 22**
**CH-4417 Ziefen(CH)**
Erfinder: **Hochuli, Erich, Dr.**
**Kirchackerstrasse 25**
**CH-4411 Arisdorf(CH)**
Erfinder: **Schacher, Alfred**
**Im Niederholzboden 58**
**CH-4125 Riehen(CH)**

(74) Vertreter: **Notegen, Eric-André et al**
**Grenzacherstrasse 124 Postfach 3255**
**CH-4002 Basel (CH)**

EP 0 339 389 B1

## Beschreibung

Zum Nachweis verschiedener viraler Antikörper, insbesondere zum Nachweis von Antikörpern gegen HIV, werden Kugeln eingesetzt, die mit gentechnologisch hergestelltem Virus-Antigen beschichtet sind. Im Falle, dass Antikörper gegen das fragliche Virus vorhanden sind, reagieren die Antikörper mit den auf der Kugel vorhandenen Virus-Antigenen. Für den Nachweis des Immunkomplexes wird nach Waschen ein Antihumanantikörper eingesetzt, der mit einer geeigneten Markierung versehen ist, insbesondere mit Peroxydase. Dieser Antihumanantikörper bindet an den zu suchenden Antikörper.

Bislang hat man die gentechnologisch hergestellten Virus-Antigene adsorptiv an die Kugel gebunden, was bedeutet, dass die Art der Bindung nicht vorhersehbar ist, so dass die Exposition der Epitope dem Zufall überlassen ist.

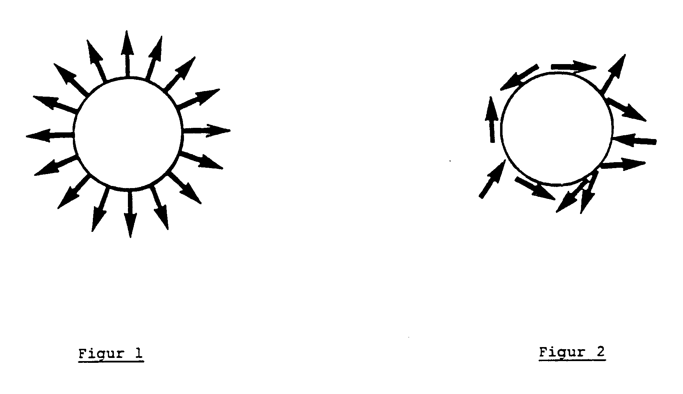

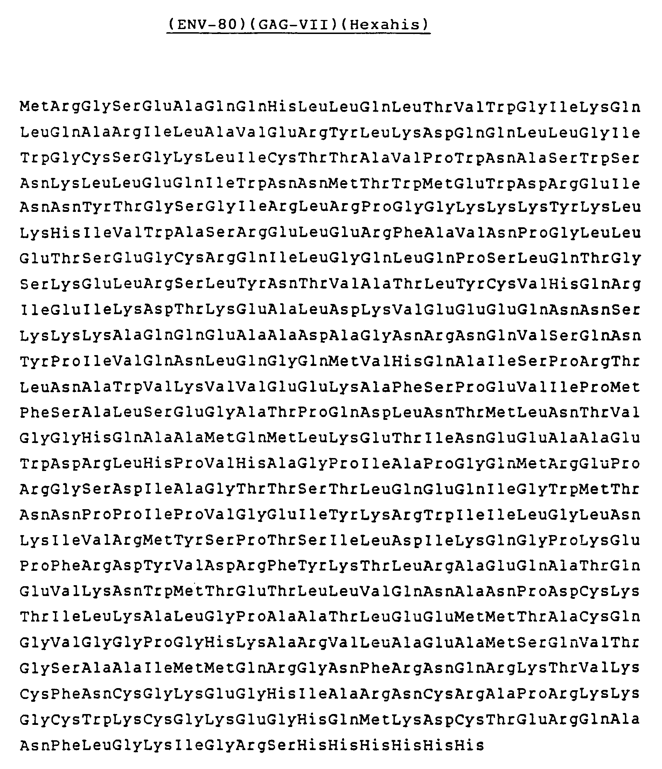

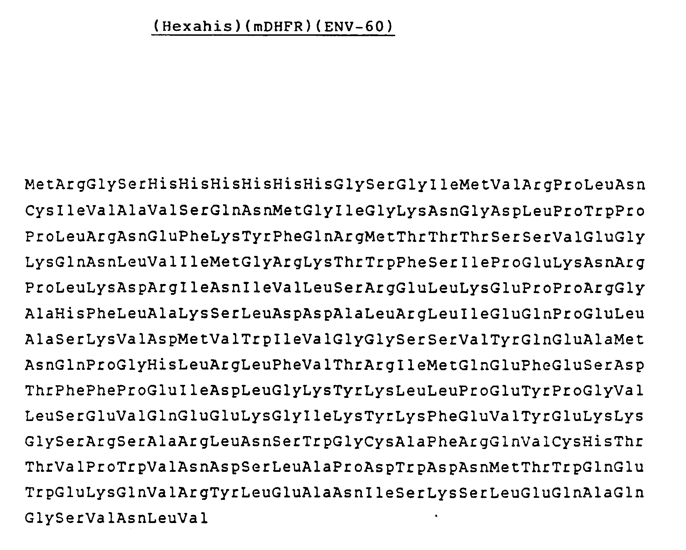

Die vorliegende Erfindung betrifft nun ein Diagnose-Hilfsmittel, im speziellen eine Festphase, an die Antigene und damit auch deren Epitope in einer wohldefinierter Weise gebunden sind (vgl. Figur 1). Dies im Gegensatz zu Festphasen, an die Antigene und damit auch deren Epitope durch Adsorption rein zufällig gebunden sind und sich teilweise überlappen (vgl. Figur 2). Die Anordnung der Antigene in definierter Weise an die Festphase erfolgt dadurch, dass gentechnologisch hergestellte Antigene mit benachbarten Histidingruppen am N- oder C-Terminus über ein mit Metallionen beladenes Nitrilotriessigsäurederivat an die Festphase gebunden sind.

Die vorerwähnten Nitrilotriessigsäurederivate entsprechen vorzugsweise der Formel $NH_2$-$(CH_2)_x$-CH-(COOH)-$N(CH_2COO^-)_2 Me^{2+}$, worin x 2, 3 oder 4 und $Me^{2+}$ ein Metallion bedeutet.

Als Metallionen kommen vorzugsweise $Co^{2+}$, $Ni^{2+}$, $Cu^{2+}$ oder $Zn^{2+}$ in Frage.

Die obenerwähnten, mit Metallionen beladenen Nitrilotriessigsäurederivate sind in Journal of Chromatography, 4.11 (1987) 177 - 184 beschrieben.

Vorzugsweise wird eine Spacer-Gruppe zwischen die Festphase und das Nitrilotriessigsäurederivat eingefügt. Die SpacerGruppen entsprechen denjenigen, die in der Affinitätschromatographie verwendet werden.

Als Festphasen kommen Materialien in Frage, wie sie in der Affinitäts- und Gelchromatographie verwendet werden, beispielsweise vernetzte Dextrane, Agarose insbesondere in der unter dem Warenzeichen Sepharose bekannten Form) oder Polyacrylamide. Besonders geeignet sind jedoch die in der Diagnostik üblicherweise verwendeten Glaskugeln, wobei diejenigen besonders bevorzugt sind, welche bereits Epoxydfunktionen an der Oberfläche aufweisen.

Durch die erfindungsgemässe Ausrichtung der Proteinantigene, wird die Bestimmung von verschiedenen viralen Antikörpern in biologischen Körperflüssigkeiten überraschenderweise wesentlich verbessert.

Die Diagnosehilfsmittel gemäss vorliegender Erfindung eignen sich vorzüglich zur Bestimmung von Antikörpern gegen HIV I, HIV II, HIV I und HIV II, Hepatitis B-Virus, Rabies-Virus, HTLV I und HTLV II.

Dem Gentechnologen ist klar, wie man z.B. in E.coli die zur Diskussion stehenden Antigene exprimieren muss, damit sie benachbarte Histidin-Gruppen am N- oder C-Terminus aufweisen.

Im weiteren betrifft die Erfindung die Verwendung der Diagnose-Hilfsmittel zum Nachweisen von Antikörpern gegen verschiedene virale Antigene, insbesondere von HIV I, HIV II, HIV I und HIV II, Hepatitis B-Virus, Rabies- Virus, HTLV I und HTLV II.

Die nachfolgenden Beispiele erläutern die Erfindung:

Beispiel 1:

Herstellung der Kugeln

Für die Herstellung der Kugeln wurden im Handel erhältliche Kugeln, welche an der Oberfläche bereits Epoxyd-Funktionen haben, verwendet.

In einem Rundkolben wurden, 1,2 g N-[5-Amino-1-Carboxypentyl]-iminodiessigsäure $H_2N(CH_2)_4$ CH-(COOH)-N-$(CH_2COOH)_2$ gelöst in 25 ml Wasser, 1,3 g $Na_2 CO_3$ und 100 Eupergit® CB 6200 Kugeln (Röhm Pharma, Darmstadt) gegeben. Das Reaktionsgemisch wurde im Wasserbad bei 55°C während 15 Stunden inkubiert. Anschliessend wurden die Kugeln in eine Chromatographiesäule gefüllt und mit Wasser neutralgewaschen. Anschliessend wurden sie zuerst mit 10 ml $CuSO_4 \cdot 6H_2O$ (2 Gew.-%) und dann mit 250 ml Wasser gewaschen. Die Kupferionenkonzentration der resultierenden Kugeln der Formel:

Eupergit® CB - CH-(OH)-$CH_2$-NH-$(CH_2)_4$ CH(COOH)-N-$(CH_2 COO-)_2 Cu^{2+}$
betrug etwa 3,5 Nanomol/Kugel.

Die Kugeln wurden wie in den nächsten Beispielen beschrieben mit HIV-Antigenen beladen.

Beispiel 2:

Coating der Kugeln mit AIDS-Antigenen

Je 100 Kugeln werden in 15 ml 6 M Guanidin-HCl pH 8 + 0,1 M Natriumphosphat gewaschen (3x). Anschliessend werden die folgenden Antigene in 15 ml des gleichen Puffers gelöst:
a) 0,26 mg (ENV-80)(GAG-VII)(Hexahis)(Aminosäuresequenz vgl. Zeichnung 2/3)
b) 0,26 mg (ENV-80)(GAG-VII)(Hexahis) + 0,55 mg (Hexahis)(mDHFR)(ENV-60)(Aminosäuresequenz vgl. Zeichnung 3/3)

Es wird während 18 Std. bei 22°C auf einer Rotationsschüttelmaschine (New Brunswick) mässig geschüttelt. Anschliessend werden die überschüssigen Antigene abgesaugt und durch zweimaliges Waschen mit je 15 ml des oben erwähnten Puffers sowie durch dreimaliges Waschen mit 0,1 M Natriumphosphat pH7 vollständig entfernt. Die Kugeln können in diesem Puffer (= feucht) oder nach Trocknen im Vakuum (= trocken) aufbewahrt werden.

Beispiel 3:

Nachweis von AIDS-Antikörpern in Humanseren

Für den Test auf AIDS-Antikörper in Humanseren können die Vorschrift sowie sämtliche Reagenzienlösungen des kommerziell erhältlichen Roche-AIDS-Diagnosticakits verwendet werden (vgl. hierzu den Prospekt Anti-HIV (HTLV III/LAV) EIA <Roche> Test, Ausgabe April 1987). Sowohl Kugeltyp a) als auch Kugeltyp b) gemäss Beispiel 2 diskriminiert klar zwischen AIDS-positiven und AIDS-negativen Seren.

## Beispiel 4

Absorptionswerte bei 492 nm (Mittelwert aus zwei Einzelbestimmungen)

| Identifikation des humanen Serums | Kugeltyp a) | Kugeltyp b) |
|---|---|---|
| NY 1, unverdünnt | 3.030 | 2.895 |
| NY 1, 1/10 verdünnt | 1.385 | 1.264 |
| NY 1, 1/100 verdünnt | 0.396 | 0.404 |
| NY 2, unverdünnt | 1.698 | 1.530 |
| Mil 3, 1/10 verdünnt | 0.842 | 0.733 |
| Mil 3, 1/30 verdünnt | 0.395 | 0.426 |
| E 6, unverdünnt | 0.209 | 0.252 |
| E 22, unverdünnt | 0.176 | 0.197 |

AIDS-positive Seren : NY 1, NY 2, Mil 3

AIDS-negative Seren : E 6, E 22

**Patentansprüche**

1. Diagnosehilfsmittel, dadurch gekennzeichnet, dass an eine Festphase, gegebenenfalls über eine Spacergruppe, über ein mit Metallionen beladenes Nitrilotriessigsäurederivat ein gentechnologisch hergestelltes Antigen mit benachbarten Histidin-Gruppen gebunden ist.

2. Diagnosehilfsmittel gemäss Anspruch 1, dadurch gekennzeichnet, dass das mit Metallionen beladene Nitrilotriessigsäurederivat der Formel

$$NH_2-(CH_2)_x-CH(COOH)-N(CH_2COO^-)_2 Me^{2+} \qquad I$$

worin x 2, 3 oder 4 bedeutet und $Me^{2+}$ ein Metallion bedeutet, entspricht.

4

**3.** Diagnosehilfsmittel gemäss Anspruch 1 oder 2, dadurch gekennzeichnet, dass $Me^{2+}$ $Co^{2+}$, $Ni^{2+}$, $Cu^{2+}$ oder $Zn^{2+}$ bedeutet.

**4.** Diagnosehilfsmittel gemäss einem der Ansprüche 1 - 3, dadurch gekennzeichnet, dass die Festphase eine in der Diagnostik übliche Glaskugel darstellt.

**5.** Diagnosehilfsmittel gemäss Anspruche 4, dadurch gekennzeichnet, dass die Kugeln an der Oberfläche Epoxydfunktionen enthalten.

**6.** Diagnosehilfsmittel gemäss einem der Ansprüche 1 - 5, dadurch gekennzeichnet, dass das gentechnologisch hergestellte Antigen ein HIV I-Antigen ist.

**7.** Diagnosehilfsmittel gemäss einem der Ansprüche 1 - 5, dadurch gekennzeichnet, dass das gentechnologisch hergestellte Antigen ein HIV II-Antigen ist.

**8.** Diagnosehilfsmittel gemäss einem der Ansprüche 1 - 5, dadurch gekennzeichnet, dass das gentechnologisch hergestellte Antigen ein HIV I- und HIV II-Antigen ist.

**9.** Diagnosehilfsmittel gemäss einem der Ansprüche 1 - 5, dadurch gekennzeichnet, dass das gentechnologisch hergestellte Antigen ein Hepatitis B-Virus-Antigen ist.

**10.** Diagnosehilfsmittel gemäss einem der Ansprüche 1 - 5, dadurch gekennzeichnet, dass das gentechnologisch hergestellte Antigen ein Rabies-Virus-Antigen ist.

**11.** Diagnosehilfsmittel gemäss einem der Ansprüche 1 - 5, dadurch gekennzeichnet, dass das gentechnologisch hergestellte Antigen ein HTLV I-Antigen ist.

**12.** Diagnosehilfsmittel gemäss einem der Ansprüche 1 - 5, dadurch gekennzeichnet, dass das gentechnologisch hergestellte Antigen ein HTLV II-Antigen ist.

**13.** Verwendung eines Diagnosehilfsmittels gemäss einem der Ansprüche 1 - 12 zum Nachweis von Antikörper gegen virale Antigene.

**14.** Verfahren zum Nachweisen von Antikörpern gegen virale Antigene, dadurch gekennzeichnet, dass man ein Diagnosehilfsmittel gemäss einem der Ansprüche 1 - 12 verwendet.

**Claims**

**1.** A diagnostic tool, characterized in that an antigen produced by gene technology and having neighbouring histidine groups is bonded to a solid phase, optionally via a spacer group, via a nitrilotriacetic acid derivative which is loaded with metal ions.

**2.** A diagnostic tool in accordance with claim 1, characterized in that the nitrilotriacetic acid derivative which is loaded with metal ions corresponds to the formula

$$NH_2\text{-}(CH_2)_x\text{-}CH(COOH)\text{-}N(CH_2COO^-)Me^{2+} \qquad I$$

wherein x signifies 2, 3 or 4 and $Me^{2+}$ signifies a metal ion.

**3.** A diagnostic tool in accordance with claim 1 or 2, characterized in that $Me^{2+}$ signifies $Co^{2+}$, $Ni^{2+}$, $Cu^{2+}$ or $Zn^{2+}$.

**4.** A diagnostic tool in accordance with any one of claims 1-3, characterized in that the solid phase is a glass bead which is conventional in diagnostics.

**5.** A diagnostic tool in accordance with claim 4, characterized in that the beads contain epoxide functions on the surface.

**6.** A diagnostic tool in accordance with any one of claims 1-5, characterized in that the antigen produced by gene technology is a HIV I antigen.

**7.** A diagnostic tool in accordance with any one of claims 1-5, characterized in that the antigen produced by gene technology is a HIV II antigen.

**8.** A diagnostic tool in accordance with any one of claims 1-5, characterized in that the antigen produced by gene technology is a HIV I and HIV II antigen.

**9.** A diagnostic tool in accordance with any one of claims 1-5, characterized in that the antigen produced by gene technology is a hepatitis B virus antigen.

**10.** A diagnostic tool in accordance with any one of claims 1-5, characterized in that the antigen produced by gene technology is a rabies virus antigen.

**11.** A diagnostic tool in accordance with any one of claims 1-5, characterized in that the antigen produced by gene technology is a HTLV I antigen.

**12.** A dianositic tool in accordance with any one of claims 1-5, characterized in that the antigen produced by gene technology is an HTLV II antigen.

**13.** A diagnostic tool in accordance with any one of claims 1-4 for the detection of antibodies against viral antigens.

**14.** A method for the detection of antibodies aganist viral antigens, characterized by using a diagnositc tool in accordance with anyone of claims 1-12.

**Revendications**

**1.** Moyen auxiliaire de test, caractérisé en ce qu'est lié à une phase solide, éventuellement par l'intermédiaire d'un groupe espaceur, par un dérivé d'acide nitrilotriacétique chargé d'ions métalliques, un antigène préparé par génie génétique ayant des groupes histidine vicinaux.

**2.** Moyen auxiliaire de test selon la revendication 1, caractérisé en ce que le dérivé d'acide nitrilotriacétique chargé d'ions métalliques correspond à la formule

$$NH_2\text{-}(CH_2)_x\text{-}CH(COOH)\text{-}N(CH_2COO^-)_2\,ME^{2+} \qquad I$$

où x est 2, 3 ou 4 et $Me^{2+}$ représente un ion métallique.

**3.** Moyen auxiliaire de test selon l'une des revendications 1 ou 2, caractérisé en ce que $Me^{2+}$ représente $Co^{2+}$, $Ni^{2+}$, $Cu^{2+}$ ou $Zn^{2+}$.

**4.** Moyen auxiliaire de test selon l'une quelconque des revendications 1-3, caractérisé en ce que la phase solide est une bille de verre classique en diagnostic.

**5.** Moyen auxiliaire de test selon la revendication 4, caractérisé en ce que les billes comportent à leur surface des fonctions époxyde.

**6.** Moyen auxiliaire de test selon l'une quelconque des revendications 1-5, caractérisé en ce que l'antigène préparé par génie génétique est un antigène de VIH I.

**7.** Moyen auxiliaire de test selon l'une quelconque des revendications 1-5, caractérisé en ce que l'antigène préparé par génie génétique est un antigène de VIH II.

**8.** Moyen auxiliaire de test selon l'une quelconque des revendications 1-5, caractérisé en ce que l'antigène préparé par génie génétique est un antigène de VIH I et VIH II.

**9.** Moyen auxiliaire de test selon l'une quelconque des revendications 1-5, caractérisé en ce que l'antigène préparé par génie génétique est un antigène du virus de l'hépatite B.

**10.** Moyen auxiliaire de test selon l'une quelconque des revendications 1-5, caractérisé en ce que l'antigène préparé par génie génétique est un antigène du virus de la rage.

**11.** Moyen auxiliaire de test selon l'une quelconque des revendications 1-5, caractérisé en ce que l'antigène préparé par génie génétique est un antigène de HTLV I.

**12.** Moyen auxiliaire de test selon l'une quelconque des revendications 1-5, caractérisé en ce que l'antigène préparé par génie génétique est un antigène de HTLV II.

**13.** Utilisation d'un moyen auxiliaire de test selon l'une quelconque des revendications 1-12 pour la mise en évidence d'anticorps contre des antigènes viraux.

**14.** Procédé pour la mise en évidence d'anticorps contre des antigènes viraux, caractérisé en ce qu'on utilise un moyen auxiliaire de test selon l'une quelconque des revendications 1-12.

Figur 1

Figur 2

(ENV-80)(GAG-VII)(Hexahis)


MetArgGlySerGluAlaGlnGlnHisLeuLeuGlnLeuThrValTrpGlyIleLysGln

LeuGlnAlaArgIleLeuAlaValGluArgTyrLeuLysAspGlnGlnLeuLeuGlyIle

TrpGlyCysSerGlyLysLeuIleCysThrThrAlaValProTrpAsnAlaSerTrpSer

AsnLysLeuLeuGluGlnIleTrpAsnAsnMetThrTrpMetGluTrpAspArgGluIle

AsnAsnTyrThrGlySerGlyIleArgLeuArgProGlyGlyLysLysLysTyrLysLeu

LysHisIleValTrpAlaSerArgGluLeuGluArgPheAlaValAsnProGlyLeuLeu

GluThrSerGluGlyCysArgGlnIleLeuGlyGlnLeuGlnProSerLeuGlnThrGly

SerLysGluLeuArgSerLeuTyrAsnThrValAlaThrLeuTyrCysValHisGlnArg

IleGluIleLysAspThrLysGluAlaLeuAspLysValGluGluGluGlnAsnAsnSer

LysLysLysAlaGlnGlnGluAlaAlaAspAlaGlyAsnArgAsnGlnValSerGlnAsn

TyrProIleValGlnAsnLeuGlnGlyGlnMetValHisGlnAlaIleSerProArgThr

LeuAsnAlaTrpValLysValValGluGluLysAlaPheSerProGluValIleProMet

PheSerAlaLeuSerGluGlyAlaThrProGlnAspLeuAsnThrMetLeuAsnThrVal

GlyGlyHisGlnAlaAlaMetGlnMetLeuLysGluThrIleAsnGluGluAlaAlaGlu

TrpAspArgLeuHisProValHisAlaGlyProIleAlaProGlyGlnMetArgGluPro

ArgGlySerAspIleAlaGlyThrThrSerThrLeuGlnGluGlnIleGlyTrpMetThr

AsnAsnProProIleProValGlyGluIleTyrLysArgTrpIleIleLeuGlyLeuAsn

LysIleValArgMetTyrSerProThrSerIleLeuAspIleLysGlnGlyProLysGlu

ProPheArgAspTyrValAspArgPheTyrLysThrLeuArgAlaGluGlnAlaThrGln

GluValLysAsnTrpMetThrGluThrLeuLeuValGlnAsnAlaAsnProAspCysLys

ThrIleLeuLysAlaLeuGlyProAlaAlaThrLeuGluGluMetMetThrAlaCysGln

GlyValGlyGlyProGlyHisLysAlaArgValLeuAlaGluAlaMetSerGlnValThr

GlySerAlaAlaIleMetMetGlnArgGlyAsnPheArgAsnGlnArgLysThrValLys

CysPheAsnCysGlyLysGluGlyHisIleAlaArgAsnCysArgAlaProArgLysLys

GlyCysTrpLysCysGlyLysGluGlyHisGlnMetLysAspCysThrGluArgGlnAla

AsnPheLeuGlyLysIleGlyArgSerHisHisHisHisHisHis


9

(Hexahis)(mDHFR)(ENV-60)

MetArgGlySerHisHisHisHisHisHisGlySerGlyIleMetValArgProLeuAsn
CysIleValAlaValSerGlnAsnMetGlyIleGlyLysAsnGlyAspLeuProTrpPro
ProLeuArgAsnGluPheLysTyrPheGlnArgMetThrThrThrSerSerValGluGly
LysGlnAsnLeuValIleMetGlyArgLysThrTrpPheSerIleProGluLysAsnArg
ProLeuLysAspArgIleAsnIleValLeuSerArgGluLeuLysGluProProArgGly
AlaHisPheLeuAlaLysSerLeuAspAspAlaLeuArgLeuIleGluGlnProGluLeu
AlaSerLysValAspMetValTrpIleValGlyGlySerSerValTyrGlnGluAlaMet
AsnGlnProGlyHisLeuArgLeuPheValThrArgIleMetGlnGluPheGluSerAsp
ThrPhePheProGluIleAspLeuGlyLysTyrLysLeuLeuProGluTyrProGlyVal
LeuSerGluValGlnGluGluLysGlyIleLysTyrLysPheGluValTyrGluLysLys
GlySerArgSerAlaArgLeuAsnSerTrpGlyCysAlaPheArgGlnValCysHisThr
ThrValProTrpValAsnAspSerLeuAlaProAspTrpAspAsnMetThrTrpGlnGlu
TrpGluLysGlnValArgTyrLeuGluAlaAsnIleSerLysSerLeuGluGlnAlaGln
GlySerValAsnLeuVal